(19) Europäisches Patentamt
European Patent Office
Office européen des brevets

(11) **EP 4 684 782 A1**

(12) **EUROPEAN PATENT APPLICATION**
published in accordance with Art. 153(4) EPC

(43) Date of publication:
**28.01.2026   Bulletin 2026/05**

(21) Application number: 24865709.0

(22) Date of filing: **20.08.2024**

(51) International Patent Classification (IPC):
**A61K 31/19** (2006.01)        **A61P 39/06** (2006.01)
**A61K 8/365** (2006.01)        **A61Q 19/00** (2006.01)
**A61Q 19/08** (2006.01)        **A61K 31/191** (2006.01)

(52) Cooperative Patent Classification (CPC):
**A61K 8/365; A61K 31/19; A61K 31/191;**
**A61P 39/06; A61Q 19/00; A61Q 19/08**

(86) International application number:
**PCT/KR2024/012297**

(87) International publication number:
**WO 2025/058268 (20.03.2025 Gazette 2025/12)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **12.09.2023   KR 20230120961
26.07.2024   KR 20240099303
26.07.2024   KR 20240099304**

(71) Applicant: **LG Chem, Ltd.
Seoul 07336 (KR)**

(72) Inventors:
• **PARK, Hun**
  **Daejeon 34122 (KR)**
• **JUNG, Woochul**
  **Daejeon 34122 (KR)**
• **YANG, Jungil**
  **Daejeon 34122 (KR)**
• **KANG, Donggyun**
  **Daejeon 34122 (KR)**

(74) Representative: **Plasseraud IP
104 Rue de Richelieu
CS92104
75080 Paris Cedex 02 (FR)**

(54) **COSMETIC COMPOSITION FOR ANTIOXIDATION**

(57)    The present disclosure relates to a cosmetic composition having antioxidant activity. According to the present disclosure, a cosmetic composition that has 2,2-diphenyl-1-picrylhydrazyl radical scavenging ability and thus can exhibit excellent antioxidant activity is provided.

[FIG. 1]

**Description**

**[TECHNICAL FIELD]**

CROSS-REFERENCE TO RELATED APPLICATION(S)

This application claims priority to and the benefit of Korean Patent Application No. 10-2023-0120961, filed on September 12, 2023, Korean Patent Application No. 10-2024-0099303, filed on July 26, 2024, and Korean Patent Application No. 10-2024-0099304, filed on July 26, 2024, with the Korean Intellectual Property Office, the disclosures of which are incorporated herein by reference in their entirety.

The present disclosure relates to a cosmetic composition having antioxidant activity.

**[BACKGROUND OF ART]**

Oxygen is absolutely necessary to maintain the life of biological organisms on Earth.

However, ground-state triplet oxygen, which is in a stable molecular state, is transformed into highly reactive oxygen such as superoxide radical, hydroxyl radical, hydrogen peroxide, and singlet oxygen by various factors such as reductive metabolism, enzymes in the body, chemicals, photochemical reactions, and pollutants. Active oxygen causes oxygen toxicity, which is fatal to living organisms.

It is known that reactive oxygen causes non-selective and irreversible destruction action with respect to cell constituent components such as proteins, lipids, sugars, and DNA, and results in aging and various diseases.

Normal cells also produce some free radicals and active oxygen during metabolic processes. However, antioxidant enzymes such as superoxide dismutase exist as a biophylactic mechanism against active oxygen. In addition, substances such as vitamin C and vitamin E are known to have antioxidant effects in the living body. However, when an abnormality occurs in the biophylactic mechanism or when the production of reactive oxygen exceeds the capacity of the biophylactic system, oxidative stress occurs.

Since the discovery of superoxide dismutase, large-scale research on antioxidants has been conducted. And, as it is known that active oxygen has an effect on diseases such as aging and adult diseases, research on antioxidants is being conducted actively.

However, various commercially available antioxidants have various problems such as toxicity to human body, low activity, and limited applications, and thus there is still a great demand for an antioxidant composition that is free from these problems.

**[DETAILED DESCRIPTION OF THE INVENTION]**

**[Technical Problem]**

It is an object of the present disclosure to provide a cosmetic composition having antioxidant activity.

It is another object of the present disclosure to provide a pharmaceutical composition having antioxidant activity.

**[Technical Solution]**

According to an embodiment of the present disclosure, there is provided a cosmetic composition having antioxidant activity, comprising 3-hydroxypropionic acid or a derivative thereof as an effective ingredient.

According to another embodiment of the present disclosure, there is provided use of 3-hydroxypropionic acid or a derivative thereof for the preparation of cosmetics having antioxidant activity.

According to yet another embodiment of the present disclosure, there is provided a method of protecting a skin from oxidative stress by using a cosmetic composition comprising 3-hydroxypropionic acid or a derivative thereof as an active ingredient.

According to yet another embodiment of the present disclosure, there is provided a pharmaceutical composition having antioxidant activity comprising 3-hydroxypropionic acid or a derivative thereof as an effective ingredient.

According to yet another embodiment of the present disclosure, there is provided use of 3-hydroxypropionic acid or a derivative thereof for the preparation of medicines having antioxidant activity.

According to yet another embodiment of the present disclosure, there is provided a method of protecting a body from oxidative stress using a pharmaceutical composition comprising 3-hydroxypropionic acid or a derivative thereof as an active ingredient.

Hereinafter, a cosmetic composition having antioxidant activity or a pharmaceutical composition having antioxidant activity according to specific embodiments of the present disclosure will be described in more detail.

Terms or words used in the present specification and claims should not be construed as limited to ordinary or dictionary terms, and the present disclosure should be construed with meanings and concepts that are consistent with the technical idea of the present disclosure based on the principle that the inventors may appropriately define concepts of the terms to appropriately describe their own invention in the best way.

Unless otherwise defined, all technical terms and scientific terms used in the specification have the general meaning understood by those skilled in the art to which the present disclosure belongs. The terms used in the specification are only to effectively describe a specific embodiment, but are not intended to limit the present disclosure.

While the present disclosure may be modified in various ways and take on various alternative forms, specific embodiments thereof are described in detail below. However, it should be understood that there is no intent to limit the present disclosure to the particular forms disclosed, but on the contrary, the present disclosure covers all modifications, equivalents, and alternatives falling within the spirit and scope of the present disclosure.

Singular terms used in the specification include plural terms, unless the context clearly indicates otherwise.

The terms "comprise," "include", "have", etc. are used herein to specify the presence of stated features, numbers, regions, steps, operations, elements, and/or components, but do not preclude the presence or addition of one or more other features, regions, numbers, steps, operations, elements, components, and/or groups thereof.

In describing temporal relationships, for example, when the temporal order is described using "after", "subsequent", "next", or "before", the case of a noncontinuous relationship may be included, unless "just" or "directly" is used.

As used herein, the term "derivative(s)" means a compound that has been modified from their parent organic compound by introduction of functional groups, oxidation, reduction, substitution of atoms, etc., to the extent that the structure and properties of the parent compound are not significantly changed.

As used herein, the term "comprising as an effective ingredient" means that the composition according to an embodiment of the present disclosure comprises 3-hydroxypropionic acid or a derivative thereof in an amount sufficient to achieve an antioxidant effect.

As used herein, the term "antioxidant" means protecting a body (e.g., skin) from oxidative stress. Oxidative stress occurs naturally in normal metabolic processes, but if it is generated or accumulated more than needed, it may damage DNA and cells, leading to aging and chronic diseases. An antioxidant system that removes oxidation-inducing substances exists in the body, but the oxidation process can be blocked or prevented by taking antioxidant substances.

As used herein, the term "prevention" means inhibiting the occurrence of symptoms caused by active oxygen. As used herein, the term "treatment" means alleviating or eliminating the appearance of symptoms caused by active oxygen that have already occurred. Further, as used herein, the term "amelioration" means alleviating or mitigating symptoms caused by active oxygen.

As a result of continuous research, the present inventors have found that 3-hydroxypropionic acid can exhibit excellent antioxidant effects such as preventing, alleviating, or ameliorating symptoms caused by active oxygen.

In particular, 3-hydroxypropionic acid has 2,2-diphenyl-1-picrylhydrazyl (hereinafter referred to as DPPH) radical scavenging ability, whereby a cosmetic composition comprising 3-hydroxypropionic acid or a derivative thereof as an effective ingredient can exhibit excellent antioxidant effects.

According to an embodiment of the present disclosure, a cosmetic composition having antioxidant activity, comprising 3-hydroxypropionic acid or a derivative thereof as an effective ingredient is provided.

The cosmetic composition having antioxidant activity comprises 3-hydroxypropionic acid or a derivative thereof as an effective ingredient. Preferably, the cosmetic composition having antioxidant activity comprises 3-hydroxypropionic acid as an effective ingredient.

3-Hydroxypropionic acid synthesized by a chemical method or a biological method can be applied as an active ingredient.

According to an embodiment, 3-hydroxypropionic acid obtained through a biological process such as fermenting a strain having 3-hydroxypropionic acid production ability can be preferably used as the effective ingredient. The 3-hydroxypropionic acid obtained by the above method can include a radioactive carbon isotope ($^{14}C$).

Preferably, the effective ingredient may have a radioactive carbon isotope content of 20 pMC(percent modern carbon) or more and a biocarbon content of 20 wt.% or more as measured according to ASTM D6866-21 standard. Specifically, the effective ingredient may have a radioactive carbon isotope content of 20 pMC or more, or 50 pMC or more, or 90 pMC or more, or 100 pMC or more, or 90 to 110 pMC and a biocarbon content of 20 wt.% or more, or 50 wt.% or more, or 80 wt.% or more, or 90 wt.% or more, or 95 wt.% or more, or 90 to 100 wt.% as measured according to ASTM D6866-21 standard.

The radioactive carbon isotope content (pMC) refers to a ratio of the radioactive carbon isotope($^{14}C$) contained in 3-hydroxypropionic acid to the radioactive carbon isotope($^{14}C$) of the modern reference material. For reference, the effectiveness of a nuclear test program in the 1950s is continuing and not extinguished, and thus the radioactive carbon isotope content (pMC) may be greater than 100%.

The biocarbon content represents the content of biocarbon with respect to a total carbon content included in 3-hydroxypropionic acid, which may mean that a higher value can correspond to an eco-friendly compound.

If the radioactive carbon isotope content (pMC) and biocarbon content of the 3-hydroxypropionic acid are too low, eco-friendliness can be reduced and it may not be regarded as a bio-derived material.

The radioactive isotope ($^{14}$C) can be nearly one per $1 \times 10^{12}$ carbon atoms in the earth's atmosphere and have a half-life of about 5,700 years, and it may be abundant in an upper atmosphere due to a nuclear reaction involving cosmic rays and common nitrogen ($^{14}$N).

In fossil fuels, radioactive isotopes have decayed long ago and thus a $^{14}$C ratio can be substantially zero. When bio-derived materials are used as a raw material for 3-hydroxypropionic acid or when fossil fuels are used together, a content of radioactive carbon isotopes (pMC) contained in 3-hydroxypropionic acid and a content of biocarbon can be measured according to ASTM D6866-21 standard.

For example, carbon atoms included in the compound to be measured can be made into the form of graphite or carbon dioxide gas, and then measured with a mass spectrometer, or according to a liquid scintillation spectroscopy. In this case, two isotopes can be separated using an accelerator for separating $^{14}$C ions from $^{12}$C ions, and thus the content and content ratio can be measured with a mass spectrometer.

The cosmetic composition having antioxidant activity can exhibit the DPPH radical scavenging ability due to the action of the effective ingredient. The DPPH radical expresses a dark purple color. The DPPH radical is reduced by the antioxidant effective ingredient, and the dark purple color is discolored. The performance of the antioxidant effective ingredient can be measured using such characteristics.

According to an embodiment, the cosmetic composition can contain 0.5 wt.% to 80 wt.% of the effective ingredient based on the total weight of the cosmetic composition.

Specifically, the cosmetic composition can contain 0.5 wt.% or more or 1 wt.% or more; and 80 wt.% or less, or 75 wt.% or less, or 70 wt.% or less of the effective ingredient based on the total weight of the cosmetic composition.

In order to ensure that the antioxidant effect can be sufficiently achieved by the action of the effective ingredient, the cosmetic composition preferably contains 0.5 wt.% or more or 1 wt.% or more of the effective ingredient.

However, if the effective ingredient is contained in an excessive amount, the antioxidant effect may be rather reduced or side effects may appear. Therefore, the cosmetic composition preferably contains 80 wt.% or less, or 75 wt.% or less, or 70 wt.% or less of the effective ingredient.

Preferably, the cosmetic composition may contain 0.5 wt.% to 80 wt.%, or 0.5 wt.% to 75 wt.%, or 1 wt.% to 75 wt.%, or 1 wt.% to 70 wt.% of the effective ingredient based on the total weight of the cosmetic composition.

According to an embodiment, the cosmetic composition may have a conventional cosmetic formulation in the technical field to which the present disclosure belongs. The cosmetic composition may be formulated in in a wide variety of forms, for example, including a solution, a suspension, an emulsion, a paste, a gel, a cream, a lotion, a powder, an oil, a foam, a pack, a spray, an aerosol, a balm, a solid soap, a liquid soap, a powder foundation, an emulsion foundation, or a wax foundation.

According to an embodiment, the cosmetic composition may, in addition to the effective ingredient, contain an organic solvent, a fat substance, a solubilizer, a concentrator, a gelling agent, a softener, an antioxidant, a suspending agent, a stabilizer, a forming agent, a fragrance, a surfactant, water, emulsifier, a filler, a chelating agent, preservative, vitamin, humectant, dye, pigment, hydrophilic or lipophilic activator, and the like. Further, the cosmetic composition may contain an adjuvant commonly used in the cosmetology or dermatology fields.

The cosmetic composition may further comprise a physiologically acceptable medium comprising water or a mixture of water and an organic solvent.

As an example, the organic solvent may include C1-C4 lower alcohols such as acetone, ethanol and isopropyl alcohol, alkylene glycols such as ethylene glycol and propylene glycol, ethylene glycol monomethyl ether, monoethyl ether, monobutyl ether, propylene glycol, and the like. The physiologically acceptable medium may be applied in an amount of 1 to 90 wt.% based on the total weight of the cosmetic composition.

The medium can be thickened using a thickener commonly used in cosmetics or pharmaceuticals. The thickener can be applied in an amount of 0.1 to 5 wt.% based on the total weight of the composition.

In addition, according to some embodiments of the cosmetic composition, a person skilled in the art can select adjuvants that are generally required for preparing such compositions. As an example, the adjuvants may comprise preservatives, stabilizers, pH regulators, osmotic pressure modifiers, emulsifiers, sunscreens, antioxidants, fragrances, dyes, anionic surfactants, cationic surfactants, nonionic surfactants, and amphoteric surfactants.

According to an embodiment, when the formulation of the cosmetic composition is a powder or spray, it may comprise carrier components such as lactose, talc, silica, aluminum hydroxide, calcium silicate, polyamide powder, and the like.

According to an embodiment, when the formulation of the cosmetic composition is a paste, gel, or cream, it may comprise carrier components such as animal fats, vegetable oils, waxes, paraffins, starch, tragacanth, cellulose derivatives, polyethylene glycols, silicones, bentonite, silica, talc, zinc oxide, and the like.

According to an embodiment, when the formulation of the cosmetic composition is a solution and emulsion, it may comprise carrier components such as solvent, solubilizer and emulsifier. For example, the formulation of solutions and emulsions may comprise water, ethanol, isopropanol, ethyl carbonate, ethyl acetate, benzyl alcohol, benzyl benzoate, propylene glycol, 1.3-butyleneglycol oils, glycerol fatty acid ester, polyethylene glycol or fatty acid ester of sorbitan, and the like.

According to an embodiment, when the formulation of the cosmetic composition is a suspension, it may comprise liquid diluents such as water, ethanol or propylene glycol; suspending agents such as ethoxylated isostearyl alcohols, polyoxyethylene sorbitol esters and polyoxy ethylene sorbitan esters; microcrystalline cellulose, aluminum metahydroxide, bentonite, and the like.

According to an embodiment, when the formulation of the cosmetic composition is a cleansing agent containing a surfactant, it may comprise carrier components such as an aliphatic alcohol sulfate, aliphatic alcohol ether sulfate, sulfosuccinic acid monoester, isethionate, imidazolinium derivative, sodium methyl cocoyl taurate, sodium lauroyl sarcosinate, fatty acid amide ether sulfate, alkylamidobetaine, fatty alcohol, fatty acid glyceride, fatty acid diethanolamide, vegetable oil, lanolin derivative, ethoxylated glycerol fatty acid ester, and the like.

According to another embodiment of the present disclosure, there is provided use of 3-hydroxypropionic acid or a derivative thereof for the preparation of cosmetics having antioxidant activity.

Preferably, the use of 3-hydroxypropionic acid or a derivative thereof for the preparation of the above-mentioned cosmetic composition having antioxidant activity is provided.

3-Hydroxypropionic acid has DPPH radical scavenging ability, whereby a cosmetic composition comprising 3-hydroxypropionic acid or a derivative thereof as an effective ingredient can be used for antioxidant purposes.

In the above applications, the effective ingredient, i.e., 3-hydroxypropionic acid or a derivative thereof, can be used in an amount of 0.5 wt.% to 80 wt.% based on the total weight of the cosmetic composition.

In order to ensure that the antioxidant effect can be sufficiently achieved by the action of the active ingredient, the effective ingredient is preferably used in an amount of 0.5 wt.% or more or 1 wt.% or more based on the total weight of the cosmetic composition. However, if the effective ingredient is used in an excessive amount, the antioxidant effect may be rather reduced or side effects may appear. Therefore, the effective ingredient is preferably used in an amount of 80 wt.% or less, or 75 wt.% or less, or 70 wt.% or less, based on the total weight of the cosmetic composition. Preferably, the effective ingredient may be used in an amount of 0.5 wt.% to 80 wt.%, or 0.5 wt.% to 75 wt.%, or 1 wt.% to 75 wt.%, or 1 wt.% to 70 wt.%, based on the total weight of the cosmetic composition.

According to yet another embodiment of the present disclosure, there is provided a method of protecting a skin from oxidative stress using a cosmetic composition comprising 3-hydroxypropionic acid or a derivative thereof as an effective ingredient. Specifically, a method for scavenging 2,2-diphenyl-1-picrylhydrazyl radicals using a cosmetic composition comprising 3-hydroxypropionic acid or a derivative thereof as an effective ingredient is provided.

Preferably, the method of protecting the skin from the oxidative stress can be performed by using the above-mentioned cosmetic composition having antioxidant activity. The method can be performed by applying the cosmetic composition to a human body site in need of antioxidant activities. The method can be performed with an application frequency of one to several times a day (for example, one to five times or one to three times) for one to six months, one to seven days a week.

According to yet another embodiment of the present disclosure, there is provided a pharmaceutical composition having antioxidant activity comprising 3-hydroxypropionic acid or a derivative thereof as an active ingredient.

As a result of continuous research, the present inventors have found that 3-hydroxypropionic acid can exhibit excellent antioxidant effects such as preventing or treating symptoms caused by active oxygen.

In particular, 3-hydroxypropionic acid has 2,2-diphenyl-1-picrylhydrazyl (hereinafter referred to as DPPH) radical scavenging ability, whereby a pharmaceutical composition comprising 3-hydroxypropionic acid or a derivative thereof as an active ingredient can exhibit excellent antioxidant effects.

The pharmaceutical composition having antioxidant activity comprises 3-hydroxypropionic acid or a derivative thereof as an active ingredient. Preferably, the pharmaceutical composition having antioxidant activity comprises 3-hydroxypropionic acid as an active ingredient.

3-Hydroxypropionic acid synthesized by a chemical method or a biological method can be applied as an active ingredient.

According to an embodiment, the effective ingredient may be preferably 3-hydroxypropionic acid obtained through a biological process such as fermenting a strain having 3-hydroxypropionic acid production ability. The 3-hydroxypropionic acid obtained by the above method may include a radioactive carbon isotope ($^{14}$C).

Preferably, the active ingredient may have a radioactive carbon isotope content of 20 pMC (percent modern carbon) or more and a biocarbon content of 20 wt.% or more as measured according to ASTM D6866-21 standard. Specifically, the active ingredient may have a radioactive carbon isotope content of 20 pMC or more, or 50 pMC or more, or 90 pMC or more, or 100 pMC or more, or 90 to 110 pMC, and a biocarbon content of 20 wt.% or more, or 50 wt.% or more, or 80 wt.% or more, or 90 wt.% or more, or 95 wt.% or more, or 90 to 100 wt.%, as measured in accordance with ASTM D6866-21 standard.

The pharmaceutical composition having antioxidant activity can exhibit the DPPH radical scavenging ability by the action of the effective ingredient. The DPPH radical expresses a dark purple color. The DPPH radical is reduced by the antioxidant effective ingredient, and the dark purple color is discolored. The performance of the antioxidant effective ingredient can be measured using such characteristics.

According to an embodiment, the pharmaceutical composition can contain 0.5 wt.% to 80 wt.% of the effective ingredient based on the total weight of the pharmaceutical composition.

Specifically, the pharmaceutical composition can contain 0.5 wt.% or more or 1 wt.% or more; and 80 wt.% or less, or 75 wt.% or less, or 70 wt.% or less of the effective ingredient based on the total weight of the pharmaceutical composition.

In order to ensure that the antioxidant effect can be sufficiently achieved by the action of the active ingredient, the pharmaceutical composition preferably contains 0.5 wt.% or more or 1 wt.% or more of the effective ingredient.

However, if the effective ingredient is included in an excessive amount, the antioxidant effect may be rather reduced or side effects may appear. Therefore, the pharmaceutical composition preferably contains 80 wt.% or less, or 75 wt.% or less, or 70 wt.% or less of the effective ingredient.

Preferably, the pharmaceutical composition may comprise 0.5 wt.% to 80 wt.%, or 0.5 wt.% to 75 wt.%, or 1 wt.% to 75 wt.%, or 1 wt.% to 70 wt.% of the effective ingredient based on the total weight of the pharmaceutical composition.

The pharmaceutical composition may further comprise a pharmaceutically acceptable carrier.

As the carrier, those commonly used in the preparation of the pharmaceutical composition may be applied without particular limitation. As an example, the carrier may be sucrose, sorbitol, mannitol, lactose, dextrose, starch, gum acacia, calcium phosphate, alginate, gelatin, calcium silicate, microcrystalline cellulose, polyvinylpyrrolidone, cellulose, water, syrup, methyl cellulose, propyl 4-hydroxybenzoate, talc, magnesium stearate, mineral oil, and the like. Pharmaceutically acceptable carriers and formulations are described in detail in Remington's The Science and Practice of Pharmacy (23rd Edition, 2020).

The pharmaceutical composition may be orally or parenterally administered. In case of the parenteral administration, this may be administered through an application to skin, an intravenous injection, a subcutaneous injection, an intramuscular injection, an intraperitoneal injection, a dermal administration, etc.

An appropriate dosage of the pharmaceutical composition may be determined in comprehensive consideration of factors such as a patient's age, weight, gender, a formulation method, an administration method, administration route, and administration time.

The pharmaceutical composition can be formulated by applying a pharmaceutically acceptable carrier and/or excipient. The formulation of the pharmaceutical composition may be a solution, suspension, emulsion, ointment, powder, granule, tablet, or capsule in an aqueous medium or oil.

According to yet another embodiment of the present disclosure, there is provided use of 3-hydroxypropionic acid or a derivative thereof for the preparation of medicines having antioxidant activity.

Preferably, the use of 3-hydroxypropionic acid or a derivative thereof for the preparation of the pharmaceutical composition having antioxidant activity is provided.

3-Hydroxypropionic acid has DPPH radical scavenging ability, whereby a pharmaceutical composition comprising 3-hydroxypropionic acid or a derivative thereof as an active ingredient can be used for antioxidant purposes.

In the above applications, the effective ingredient, i.e., 3-hydroxypropionic acid or a derivative thereof, can be used in an amount of 0.5 wt.% to 80 wt.% based on the total weight of the pharmaceutical composition.

In order to ensure that the antioxidant effect can be sufficiently achieved by the action of the active ingredient, the effective ingredient is preferably used in an amount of 0.5 wt.% or more or 1 wt.% or more based on the total weight of the pharmaceutical composition. However, if the effective ingredient is used in an excessive amount, the antioxidant effect may be rather reduced or side effects may appear. Therefore, the effective ingredient is preferably used in an amount of 80 wt.% or less, or 75 wt.% or less, or 70 wt.% or less, based on the total weight of the pharmaceutical composition. Preferably, the effective ingredient may be used in an amount of 0.5 wt.% to 80 wt.%, or 0.5 wt.% to 75 wt.%, or 1 wt.% to 75 wt.%, or 1 wt.% to 70 wt.%, based on the total weight of the pharmaceutical composition.

According to yet another embodiment of the present disclosure, there is provided a method of protecting a body from oxidative stress using a pharmaceutical composition comprising 3-hydroxypropionic acid or a derivative thereof as an effective ingredient. Specifically, a method for scavenging 2,2-diphenyl-1-picrylhydrazyl radicals using a pharmaceutical composition comprising 3-hydroxypropionic acid or a derivative thereof as an effective ingredient is provided.

Preferably, the method of protecting the body from the oxidative stress can be performed by using the above-mentioned pharmaceutical composition having antioxidant activity. The method can be performed orally or parenterally. The method can be performed with an application frequency of one to several times a day (for example, one to five times or one to three times) for one to six months, one to seven days a week. The method may be performed according to a conventional procedure in comprehensive consideration of factors such as a patient's age, weight, gender, formulation of 3-hydroxypropionic acid or a derivative thereof, an administration method, and administration time.

[ADVANTAGEOUS EFFECTS]

According to the present disclosure, a cosmetic composition and a pharmaceutical composition having 2,2-diphenyl-1-picrylhydrazyl radical scavenging ability and thus exhibiting excellent antioxidant effects are provided.

[BRIEF DESCRIPTION OF THE DRAWING]

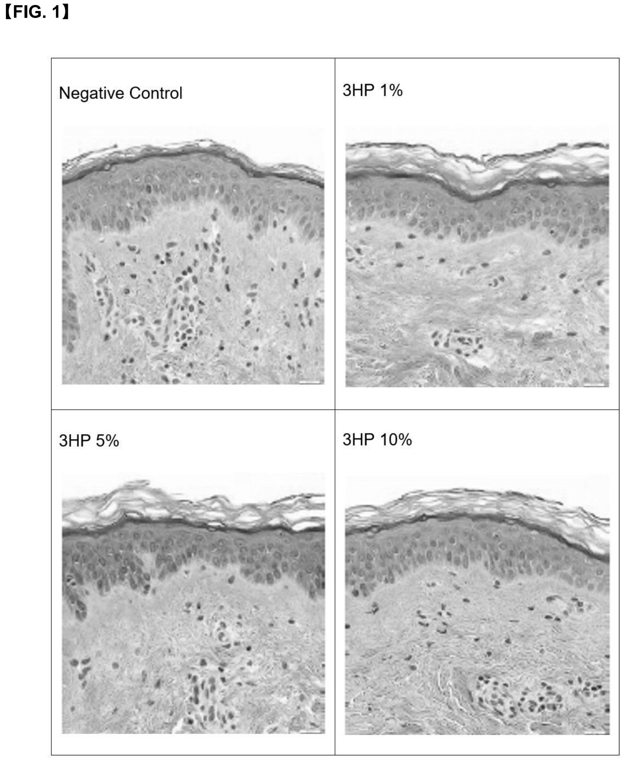

FIG. 1 is an image showing the results of a skin tissue cell stability experiment according to Experimental Example 2.

[DETAILED DESCRIPTION OF THE EMBODIMENTS]

Hereinafter, actions and effects of the invention will be described in more detail with reference to specific examples. However, the following examples are provided to assist the understanding of the invention, and are not intended to limit the scope of the invention in any way. It would be obvious to those skilled in the art that various changes and modifications can be made without departing from the sprit and scope of the invention.

Preparation Example 1

Prepared was a recombinant vector incorporating a gene encoding glycerol dehydratase and aldehyde dehydrogenase, which are known to produce 3-hydroxypropionic acid using glycerol as a substrate. The prepared recombinant vector was introduced into the *E. coli* W3110 strain to prepare a 3-hydroxypropionic acid-producing strain.

Specifically, pCDF_J23101_dhaB_gdrAB_J23100_aldH_btuR vector, was obtained by cloning BtuR gene encoding adenosyltransferase into plasmid pCDF including a gene(dhaB) encoding glycerol dehydratase, a gene(aldH) encoding aldehyde dehydrogenase and a gene(gdrAB) encoding glycerol dehydratase reactivase. The vector was introduced into W3110 strain (KCCM 40219) by electroporation using an electroporation device (Bio-Rad, Gene Pulser Xcell) to prepare a 3-hydroxypropionic acid-producing strain.

The 3-hydroxypropionic acid-producing strain was fermented and cultured at 35°C in a 5 L fermenter by using unpurified glycerol as a carbon source, so as to produce 3-hydroxypropionic acid. In order to prevent a decrease in pH due to the production of 3-hydroxypropionic acid, calcium hydroxide ($Ca(OH)_2$), an alkali metal salt, was added to maintain a neutral pH during fermentation.

After fermentation culture, cells were removed by centrifugation (4000 rpm, 10 minutes, 4°C), and primary purification was performed by using activated carbon. The concentration of 3-hydroxypropionic acid in the fermented solution obtained after the primary purification was at a level of 50 to 100 g/L, and the fermented solution was concentrated at a concentration of 600 g/L using a rotary evaporator (50°C, 50 mbar) to prepare a concentrate, which was then stirred at room temperature (300 rpm) to produce $Ca(3HP)_2$ crystals.

The resulting crystals were washed three times with ethanol and dried in an oven at 50°C to finally recover crystals. The weight of the crystals was measured, the crystals were dissolved in water to obtain 3-hydroxypropionic acid(3HP) using high performance liquid chromatography(HPLC).

Preparation Example 2

3-Hydroxypropionic acid(3HP) was obtained in substantially the same manner as in Preparation Example 1, except for using $Mg(OH)_2$ instead of $Ca(OH)_2$ as an alkali metal salt.

Example 1

1 wt.% of 3-hydroxypropionic acid obtained from Preparation Example 1, 0.5 wt.% of hydroxyethyl cellulose, 2 wt.% of cetostearyl alcohol, 2 wt.% of stearyl triethyl ammonium chloride, and the remaining amount of purified water (total 100 wt.%) were mixed to prepare a lotion.

Example 2

5 wt.% of 3-hydroxypropionic acid obtained from Preparation Example 1, 0.5 wt.% of hydroxyethyl cellulose, 2 wt.% of cetostearyl alcohol, 2 wt.% of stearyl triethyl ammonium chloride, and the remaining amount of purified water (total 100 wt.%) were mixed to prepare a lotion.

Example 3

1 wt.% of 3-hydroxypropionic acid obtained from Preparation Example 1, 55 wt.% of ethanol, 3 wt.% of glycerin, 5 wt.% of castor oil, and the remaining amount of purified water (total 100 wt.%) were mixed to prepare a toner emulsion.

Example 4

5 wt.% of 3-hydroxypropionic acid obtained from Preparation Example 1, 55 wt.% of ethanol, 3 wt.% of glycerin, 5 wt.% of castor oil, and the remaining amount of purified water (total 100 wt.%) were mixed to prepare a toner emulsion.

Example 5

10 wt.% of 3-hydroxypropionic acid obtained from Preparation Example 1, 55 wt.% of ethanol, 3 wt.% of glycerin, 5 wt.% of castor oil, and the remaining amount of purified water (total 100 wt.%) were mixed to prepare a toner emulsion.

Example 6

1 wt.% of 3-hydroxypropionic acid obtained from Preparation Example 1, 0.5 wt.% of hydroxyethyl cellulose, 2 wt.% of cetostearyl alcohol, 2 wt.% of stearyl triethyl ammonium chloride, and the remaining amount of purified water (total 100 wt.%) were mixed to prepare a pharmaceutical composition as an ointment formulation.

Example 7

5 wt.% of 3-hydroxypropionic acid obtained from Preparation Example 1, 0.5 wt.% of hydroxyethyl cellulose, 2 wt.% of cetostearyl alcohol, 2 wt.% of stearyl triethyl ammonium chloride, and the remaining amount of purified water (total 100 wt.%) were mixed to prepare a pharmaceutical composition as an ointment formulation.

Example 8

1 wt.% of 3-hydroxypropionic acid obtained from Preparation Example 1, 55 wt.% of ethanol, 3 wt.% of glycerin, 5 wt.% of castor oil, and the remaining amount of purified water (total 100 wt.%) were mixed to prepare a pharmaceutical composition as an emulsion formulation.

Example 9

5 wt.% of 3-hydroxypropionic acid obtained from Preparation Example 1, 55 wt.% of ethanol, 3 wt.% of glycerin, 5 wt.% of castor oil, and the remaining amount of purified water (total 100 wt.%) were mixed to prepare a pharmaceutical composition as an emulsion formulation.

Example 10

10 wt.% of 3-hydroxypropionic acid obtained from Preparation Example 1, 55 wt.% of ethanol, 3 wt.% of glycerin, 5 wt.% of castor oil, and the remaining amount of purified water (total 100 wt.%) were mixed to prepare a pharmaceutical composition as an emulsion formulation.

Experimental Example 1

(Measurement of radioactive carbon isotope and biocarbon content)

The radioactive carbon isotope content(pMC) and biocarbon content of the 3-hydroxypropionic acid obtained from Preparation Examples 1 and 2 were measured according to ASTM D6866-21 (Method B).
The biocarbon content means the content of biocarbon contained in the 3-hydroxypropionate crystals obtained from Preparation Examples 1 and 2, and the radioactive carbon isotope ratio(pMC) refers to a ratio of the radioactive carbon isotope ($^{14}$C) contained in the 3-hydroxypropionate crystals to the radioactive carbon isotope ($^{14}$C) of the modern reference material.

[Table 1]

| | Concentration (g/L) | Radioactive carbon isotope ratio (pMC) | Biocarbon content (wt.%) |
|---|---|---|---|
| Preparation Example 1 | 600 | 101.52 | 100 |
| Preparation Example 2 | 800 | 102.23 | 100 |

Referring to Table 1, it was confirmed that both of the 3HP salt crystals of Preparation Examples 1 and 2 have a radioactive carbon isotope ratio of 101 pMC or more and a biocarbon content of 100 wt.%.

Experimental Example 2

(Skin tissue cell stability experiment)

1) Explant tissue culture

The human skin tissues provided for research purposes (IRB No. 4-2023-0054; Institutional Review Board, Severance Medical Center, Yonsei University College of Medicine) was defatted, and washed several times with PBS (phosphate buffered saline; Lonza) to remove residual impurities. The skin tissues were prepared into a size of 1 cm $\times$ 1 cm for each test group, and the test composition was applied to the prepared skin tissues, and PBS was applied to the negative control. The skin tissues in which the absorption of the test composition has been completed were cultured in a special medium at 37°C, 5% $CO_2$.

2) HE (Hematoxylin eosin) staining

Tissues obtained 24 hours after application of the test composition were fixed with 10% formalin, and then paraffin blocks and paraffin-embedded slides were prepared. The deparaffinized tissues were stained for nuclei with hematoxylin (S3309; dako) solution, and then washed in running water. The cytoplasm was stained with eosin (318906; sigma) solution, and then the tissues were completely dehydrated through washing process and fixed using a mounting solution. The epidermis and papillary dermis of the fixed tissues were photographed at 400x magnification using an optical microscope (BX43F; OLYMPUS).
Referring to FIG.1, it was confirmed that no damage to the epidermis and dermis or separation between tissues were observed in the skin tissue to which 3HP was applied at concentrations of 1%, 5%, and 10%.

Experimental Example 3 (Control group)

The 3HP aqueous solution obtained from Preparation Example 1 was prepared at five different concentrations (0 wt.%, 1 wt.%, 5 wt.%, 10 wt.%, 70 wt.%), 50 $\mu\ell$ of each 3HP aqueous solution and 150 $\mu\ell$ of methanol were dispensed into a 96-well plate and reacted at room temperature for 30 minutes. The absorbance ($OD_{control\ group}$, wavelength 520 nm) was repeatedly measured three times.

Experimental Example 4 (Negative control group)

A DPPH aqueous solution containing 0.5 mM DPPH (free radical, 95%, Alfa Aesar) was prepared. 50 $\mu\ell$ of methanol and 150 $\mu\ell$ of the DPPH aqueous solution were dispensed into a 96-well plate and reacted at room temperature for 30 minutes. The absorbance ($OD_{negative\ control\ group}$, wavelength 520 nm) was repeatedly measured three times.

Experimental Example 5 (Experimental group)

A DPPH aqueous solution containing 0.5 mM DPPH (free radical, 95%, Alfa Aesar) was prepared. The 3HP aqueous solution obtained from Preparation Example 1 was prepared at five different concentrations (0 wt.%, 1 wt.%, 5 wt.%, 10 wt.%, 70 wt.%), and 50 $\mu\ell$ of each 3HP aqueous solution and 150 $\mu\ell$ of the DPPH aqueous solution were dispensed into a 96-well plate and reacted at room temperature for 30 minutes. The absorbance ($OD_{experimental\ group}$, wavelength 520 nm) was repeatedly measured three times.
The absorbance at a wavelength of 520 nm was measured using a micro reader.
The DPPH radical scavenging ability (antioxidant power) of 3HP was calculated according to the following Equation 1, and the results are shown in Tables below.

Antioxidant power(%) = {[(ODnegative control group- ODcontrol group) - (ODexperimental group - ODcontrol control)] / (ODnegative control group- ODcontrol control)} X 100 [Equation 1]

[Table 2]

| 3HP treated concentration | ODcontrol group | | | | |
|---|---|---|---|---|---|
| | Once | Two times | Three times | Average | Standard deviation |
| 0 % | 0.039 | 0.039 | 0.039 | 0.039 | 0.000 |
| 1 % | 0.039 | 0.039 | 0.039 | 0.039 | 0.000 |
| 5% | 0.041 | 0.042 | 0.043 | 0.042 | 0.001 |
| 10 % | 0.040 | 0.040 | 0.040 | 0.040 | 0.000 |
| 70 % | 0.037 | 0.036 | 0.037 | 0.037 | 0.000 |

[Table 3]

| 3HP treated concentration | $OD_{experimental\ group}$ | | | | |
|---|---|---|---|---|---|
| | Once | Two times | Three times | Average | Standard deviation |
| 0% | 0.616 | 0.614 | 0.613 | 0.614 | 0.001 |
| 1 % | 0.610 | 0.609 | 0.608 | 0.609 | 0.001 |
| 5% | 0.602 | 0.600 | 0.599 | 0.600 | 0.001 |
| 10 % | 0.586 | 0.585 | 0.584 | 0.585 | 0.001 |
| 70 % | 0.495 | 0.492 | 0.492 | 0.493 | 0.002 |

[Table 4]

| 3HP treated concentration | ODnegative control group | | | | |
|---|---|---|---|---|---|
| | Once | Two times | Three times | Average | Standard deviation |
| 0% | 0.616 | 0.614 | 0.613 | 0.614 | 0.001 |

[Table 5]

| 3HP treated concentratio n | Antioxidant power(%) | | | | | |
|---|---|---|---|---|---|---|
| | Once | Two times | Three times | Average | Standard deviation | Significance probability relative to 3HP 0% (p-value) |
| 0 % | 0.000 | 0.000 | 0.000 | 0.000 | 0.000 | - |
| 1 % | 0.850 | 1.042 | 0.819 | 0.904 | 0.121 | 0.006 |
| 5% | 2.452 | 2.552 | 2.419 | 2.474 | 0.069 | 0.000 |
| 10 % | 5.209 | 5.205 | 5.111 | 5.175 | 0.056 | 0.000 |
| 70 % | 20.888 | 21.142 | 21.002 | 21.011 | 0.127 | 0.000 |

Referring to the experimental results, it was confirmed that significant DPPH radical scavenging ability was exhibited under the treatment concentration of the 3-hydroxypropionic acid.

Formulation Example 1

0.2 wt.% of 3-hydroxypropionic acid obtained from Preparation Example 1, 3.0 wt.% of glycerin, 2.0 wt.% of butylene glycol, 2.0 wt.% of propylene glycol, 0.1 wt.% of polyacrylic acid, 0.2 wt.% of polyethylene glycol-12 nonylphenyl ether, 0.4 wt.% of polysorbate-80, 10.0 wt.% of ethanol, 0.1 wt.% of triethanolamine, and the remaining amount of purified water were mixed to prepare a softening lotion (skin lotion).

Formulation Example 2

1.0 wt.% of 3-hydroxypropionic acid obtained from Preparation Example 1, 3.0 wt.% of glycerin, 3.0 wt.% of butylene glycol, 0.5 wt.% of liquid paraffin, 4.0 wt.% of beeswax, 5.0 wt.% of caprylic/capric triglyceride, 5.0 wt.% of squalene, 1.5 wt.% of polysorbate-60, 1.0 wt.% of cetearyl alcohol, 1.5 wt.% of sorbitan sesquioleate, 0.1 wt.% of polyacrylic acid, 0.2 wt.% of triethanol amine, and the remaining amount of purified water were mixed to prepare a nourishing lotion (milk lotion).

Formulation Example 3

2.0 wt.% of 3-hydroxypropionic acid obtained from Preparation Example 1, 3.0 wt.% of glycerin, 3.0 wt.% of butylene glycol, 7.0 wt.% of liquid paraffin, 7.0 wt.% of beta-glucan, 3.0 wt.% of caprylic/capric triglyceride, 5.0 wt.% of squalene, 1.5 wt.% of cetearyl glucoside, 1.2 wt.% of polysorbate-60, 0.4 wt.% of sorbitan stearate, 0.1 wt.% of polyacrylic acid, 0.1 wt.% of triethanol amine, and the remaining amount of purified water were mixed to prepare a nourishing cream.

Formulation Example 4

8 mg of 3-hydroxypropionic acid obtained from Preparation Example 1, 9 mg of vitamin C, 9 mg of vitamin E, 200 mg of lactose, and 200 mg of galactooligosaccharide were mixed, and granulated using a fluidized bed dryer, and 5 mg of sugar ester was added to prepare a tablet composition. 500 mg of the tablet composition was tableted to prepare tablets.

Formulation Example 5

8 mg of 3-hydroxypropionic acid obtained from Preparation Example 1, 9 mg of vitamin C, 9 mg of vitamin E, 10 mg of vegetable hardened oil, and 9 mg of lecithin were mixed to prepare a soft capsule filling liquid. Separately, 65 wt.% of gelatin, 25 wt.% of glycerin, and 10 wt.% of sorbitol solution were mixed to prepare a soft capsule sheet. The soft capsule filling liquid was filled at 400 mg per capsule to prepare a soft capsule.

Formulation Example 6

8 mg of 3-hydroxypropionic acid obtained from Preparation Example 1, 9 mg of vitamin C, 9 mg of vitamin E, 500 mg of starch, and 200 mg of anhydrous crystalline glucose were mixed, formed into granules using a fluidized bed granulator, and then filled into a capsule to prepare a granule formulation.

Formulation Example 7

8 mg of 3-hydroxypropionic acid obtained from Preparation Example 1, 9 mg of vitamin C, 9 mg of vitamin E, 0.7 g of citric acid, 10 g of glucose, and 25 g of liquid oligosaccharide were mixed and then 300 ml of purified water were added to prepare a drink composition. The drink composition was filled into bottles at 200 ml each and sterilized at 130°C for about 5 seconds to prepare a drink formulation.

Formulation Example 8

30 mg of 3-hydroxypropionic acid obtained from Preparation Example 1, an appropriate amount of sterilized distilled water for injection, and an appropriate amount of a pH regulator were mixed to prepare an injection formulation.
While the present disclosure has been described with reference to limited embodiments, the present disclosure should be not limited to the disclosed embodiment and it will be apparent to those skilled in the art that various modifications and variations may be made without departing from the scope of the technical idea of the present disclosure and the scope of the appended claims and their equivalents.

**Claims**

1. A cosmetic composition having antioxidant activity, comprising 3-hydroxypropionic acid or a derivative thereof as an effective ingredient.

2. The cosmetic composition having antioxidant activity according to claim 1, wherein the cosmetic composition has 2,2-diphenyl-1-picrylhydrazyl radical scavenging ability.

3. The cosmetic composition having antioxidant activity according to claim 1, wherein the cosmetic composition comprises 0.5 wt.% to 80 wt.% of the effective ingredient based on the total weight of the composition.

4. The cosmetic composition having antioxidant activity according to claim 1, wherein the effective ingredient has a radioactive carbon isotope content of 20 pMC (percent modern carbon) or more and a biocarbon content of 20 wt.% or more as measured according to ASTM D6866-21 standard.

5. The cosmetic composition having antioxidant activity according to claim 1, wherein the cosmetic composition further comprises a physiologically acceptable medium.

6. The cosmetic composition having antioxidant activity according to claim 1, wherein the cosmetic composition has a formulation that include a solution, a suspension, an emulsion, a paste, a gel, a cream, a lotion, a powder, an oil, a foam, a pack, a spray, an aerosol, a balm, a solid soap, a liquid soap, a powder foundation, an emulsion foundation, or a wax foundation.

7. A pharmaceutical composition having antioxidant activity comprising 3-hydroxypropionic acid or a derivative thereof as an effective ingredient.

8. The pharmaceutical composition having antioxidant activity according to claim 7, wherein the pharmaceutical composition has 2,2-diphenyl-1-picrylhydrazyl radical scavenging ability.

9. The pharmaceutical composition having antioxidant activity according to claim 7, wherein the pharmaceutical composition comprises 0.5 wt.% to 80 wt.% of the effective ingredient based on the total weight of the composition.

10. The pharmaceutical composition having antioxidant activity according to claim 7, wherein the effective ingredient has a radioactive carbon isotope content of 20 pMC (percent modern carbon) or more and a biocarbon content of 20 wt.% or more as measured according to ASTM D6866-21 standard.

11. The pharmaceutical composition having antioxidant activity according to claim 7, wherein the pharmaceutical composition further comprises a pharmaceutically acceptable carrier.

12. The pharmaceutical composition having antioxidant activity according to claim 7, wherein the pharmaceutical composition is for oral or parenteral use.

13. Use of 3-hydroxypropionic acid or a derivative thereof for the preparation of cosmetics having antioxidant activity or medicines having antioxidant activity.

14. A method of protecting a body from oxidative stress by using a cosmetic composition or a pharmaceutical composition, comprising 3-hydroxypropionic acid or a derivative thereof as an active ingredient.

【FIG. 1】

## INTERNATIONAL SEARCH REPORT

| International application No. |
| --- |
| **PCT/KR2024/012297** |

### A. CLASSIFICATION OF SUBJECT MATTER

**A61K 31/19**(2006.01)i; **A61P 39/06**(2006.01)i; **A61K 8/365**(2006.01)i; **A61Q 19/00**(2006.01)i; **A61Q 19/08**(2006.01)i; **A61K 31/191**(2006.01)i

According to International Patent Classification (IPC) or to both national classification and IPC

### B. FIELDS SEARCHED

Minimum documentation searched (classification system followed by classification symbols)

A61K 31/19(2006.01); A61K 8/97(2006.01); A61Q 19/02(2006.01); C07C 51/43(2006.01); C07C 51/50(2006.01); C07C 67/48(2006.01); C07C 69/675(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 3-하이드록시프로피온산(3-hydroxypropionic acid), 항산화(antioxidant)

### C. DOCUMENTS CONSIDERED TO BE RELEVANT

| Category* | Citation of document, with indication, where appropriate, of the relevant passages | Relevant to claim No. |
| --- | --- | --- |
| X | KHLIFI, Rihab et al. Heat treatment improves the immunomodulatory and cellular antioxidant behavior of a natural flavanone: Eriodictyol. International Immunopharmacology. 2018, vol. 61, pp. 317-324.<br>See abstract; page 318, left column; page 321, left column; page 322, left column and page 323, left column; and figures 1 and 7. | 1-3,5-9,11-13 |
| Y | | 4,10 |
| Y | KR 10-2022-0103611 A (LG CHEM, LTD.) 22 July 2022 (2022-07-22)<br>See claims 1 and 10. | 4,10 |
| A | TANG, Yiqun et al. Antioxidant and cardioprotective effects of Danshensu (3-(3,4-dihydroxyphenyl)-2-hydroxy-propanoic acid from Salvia miltiorrhiza) on isoproterenol-induced myocardial hypertrophy in rats. Phytomedicine. 2011, vol. 18, pp. 1024-1030.<br>See entire document. | 1-13 |
| A | CN 106905159 A (DALI UNIVERSITY) 30 June 2017 (2017-06-30)<br>See entire document. | 1-13 |

☑ Further documents are listed in the continuation of Box C.   ☑ See patent family annex.

| | | |
| --- | --- | --- |
| \* | Special categories of cited documents: | |
| "A" | document defining the general state of the art which is not considered to be of particular relevance | "T" | later document published after the international filing date or priority date and not in conflict with the application but cited to understand the principle or theory underlying the invention |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" | document member of the same patent family |

| Date of the actual completion of the international search | Date of mailing of the international search report |
| --- | --- |
| **06 December 2024** | **06 December 2024** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

EP 4 684 782 A1

**INTERNATIONAL SEARCH REPORT**

| International application No. |
| --- |
| **PCT/KR2024/012297** |

### C.    DOCUMENTS CONSIDERED TO BE RELEVANT

| Category* | Citation of document, with indication, where appropriate, of the relevant passages | Relevant to claim No. |
| --- | --- | --- |
| A | KR 10-2010-0060711 A (AMOREPACIFIC CORPORATION) 07 June 2010 (2010-06-07)<br>See entire document. | 1-13 |

Form PCT/ISA/210 (second sheet) (July 2022)

15

## INTERNATIONAL SEARCH REPORT

| Box No. II | Observations where certain claims were found unsearchable (Continuation of item 2 of first sheet) |
|---|---|

This international search report has not been established in respect of certain claims under Article 17(2)(a) for the following reasons:

1. ☑ Claims Nos.: **14**
   because they relate to subject matter not required to be searched by this Authority, namely:

   Claim 14 pertains to a method for treatment of the human body by therapy (PCT Article 17(2)(a)(i) and PCT Rule 39.1(iv)).

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

EP 4 684 782 A1

**INTERNATIONAL SEARCH REPORT**
**Information on patent family members**

International application No.

**PCT/KR2024/012297**

| Patent document cited in search report | | | Publication date (day/month/year) | Patent family member(s) | | | Publication date (day/month/year) |
|---|---|---|---|---|---|---|---|
| KR | 10-2022-0103611 | A | 22 July 2022 | CN | 115702136 | A | 14 February 2023 |
| | | | | EP | 4148037 | A1 | 15 March 2023 |
| | | | | EP | 4148037 | A4 | 14 February 2024 |
| | | | | EP | 4428117 | A2 | 11 September 2024 |
| | | | | JP | 2023-531645 | A | 25 July 2023 |
| | | | | JP | 2024-045647 | A | 02 April 2024 |
| | | | | JP | 7483305 | B2 | 15 May 2024 |
| | | | | KR | 10-2023-0161919 | A | 28 November 2023 |
| | | | | US | 2023-0250044 | A1 | 10 August 2023 |
| | | | | WO | 2022-154537 | A1 | 21 July 2022 |
| CN | 106905159 | A | 30 June 2017 | CN | 106905159 | B | 08 October 2019 |
| KR | 10-2010-0060711 | A | 07 June 2010 | KR | 10-1017586 | B1 | 28 February 2011 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020230120961
- KR 1020240099303
- KR 1020240099304

**Non-patent literature cited in the description**

- Remington's The Science and Practice of Pharmacy. 2020